# EUROPÄISCHE PATENTSCHRIFT

(11) **EP 1 957 606 B1**
(45) Veröffentlichungstag und Bekanntmachung des Hinweises auf die Patenterteilung: **08.11.2017**
(21) Anmeldenummer: 06829379.4
(22) Anmeldetag: 07.12.2006
(51) Int. Cl.: C09K 11/06, H05B 33/14, C07C 211/54, C07C 15/28, H01L 51/00, H01L 51/50

(54) **NEUE MATERIALIEN FÜR ORGANISCHE ELEKTROLUMINIESZENZVORRICHTUNGEN**
NOVEL MATERIALS FOR ORGANIC ELECTROLUMINIESCENT DEVICES
NOUVELLES MATIERES POUR DISPOSITIFS ELECTROLUMINESCENTS ORGANIQUES

(30) Priorität: 08.12.2005 DE 102005058557
(43) Veröffentlichungstag der Anmeldung: 20.08.2008
(73) Patentinhaber: Merck Patent GmbH, 64293 Darmstadt (DE)
(72) Erfinder: HEIL, Holger, 64295 Darmstadt (DE); BUESING, Arne, 65929 Frankfurt/Main (DE); STOESSEL, Philipp, 60487 Frankfurt/Main (DE)
(86) Internationale Anmeldenummer: PCT/EP2006/011758
(87) Internationale Veröffentlichungsnummer: WO 2007/065678

(56) Entgegenhaltungen:
- EP-A- 1 553 154
- EP-A- 1 717 875
- EP-A1- 1 167 488
- EP-A1- 1 182 183
- EP-A2- 0 681 019
- WO-A-2006/117052
- JP-A- 11 111 460
- JP-A- 2000 007 604
- JP-A- 2005 008 587
- US-A1- 2003 111 692
- US-A1- 2004 263 067

## Beschreibung

Die vorliegende Erfindung betrifft neue Materialien für organische Elektrolumineszenzvorrichtungen, deren Verwendung, sowie organische Elektrolumineszenzvorrichtungen enthaltend diese Materialien.

Der allgemeine Aufbau organischer Elektrolumineszenzvorrichtungen, die zur Emission von Licht im sichtbaren Spektralbereich befähigt sind und die halbleitende organische Verbindungen enthalten, ist beispielsweise in US 4539507, US 5151629, EP 0676461 und WO 98/27136 beschrieben.

Allerdings zeigen diese Vorrichtungen immer noch erhebliche Probleme, die für die Verwendung in hochwertigen Vollfarbdisplays einer dringenden Verbesserung bedürfen:
1. Die operative Lebensdauer ist insbesondere bei blauer Emission immer noch gering, so dass bis dato nur einfache Anwendungen kommerziell realisiert werden konnten.
2. Auch die Effizienz ist insbesondere bei blauer Emission noch nicht ausreichend und muss für hochwertige Anwendungen weiter verbessert werden.
3. Einige Verbindungen, die als Hostmaterialien für blau emittierende Elektrolumineszenzvorrichtungen verwendet werden, neigen beim Aufdampfen zur Kristallisation statt zur Bildung glasartiger Filme und weisen keine ausreichend hohe Glasübergangstemperatur auf. Hier sind weitere Verbesserungen erforderlich.

Es war daher die Aufgabe der vorliegenden Erfindung, hierfür Verbesserungen anzubieten, insbesondere Verbindungen, welche in organischen Elektrolumineszenzvorrichtungen zu verbesserter Effizienz und verbesserter Lebensdauer führen. Weiterhin war es die Aufgabe der vorliegenden Erfindung, Verbindungen bereitzustellen, welche eine höhere Glasübergangstemperatur und eine geringere Kristallisationsneigung aufweisen.

Aus dem Stand der Technik sind Phenylanthracen-Derivate bekannt, in denen zwei Phenylanthracengruppen über eine bivalente Gruppe verknüpft sind (EP 0681019). Als bivalente Gruppen sind Einfachbindungen und Arylengruppen offenbart, insbesondere Phenylengruppen und Phenylengruppen, welche durch eine Alkylengruppe, -O-, -S- oder -NRunterbrochen sind. Aus dieser Anmeldung geht nicht hervor, dass andere bivalente Gruppen zur Verknüpfung der beiden Anthracen besonders geeignet sein könnten. Ebenso wenig geht aus dieser Anmeldung hervor, dass Anthracenderivate, welche statt der Phenylgruppe andere Gruppen gebunden enthalten, besonders geeignet sein könnten.
Aus dem Stand der Technik sind weiterhin dimere Anthracenderivate der Formel Anthracen-X-Anthracen bekannt, wobei X eine heterocyclische Verbindung mit mindestens zwei Ringen darstellt (JP 2004/002351). Aus dieser Anmeldung geht nicht hervor, dass derartige Anthracenderivate, welche in 9- und 9'-Position eine Heteroarylgruppe gebunden enthalten, besonders geeignet sind.
EP 1221434 offenbart Verbindungen, in denen zwei Anthraceneinheiten über eine Fluoreneinheit verknüpft sind. Unter anderem ist auch eine Verbindung offenbart, welche zusätzlich in 9- und 9'-Position der beiden Anthraceneinheiten jeweils eine Pyridingruppe enthält. Allerdings lehrt dieses Dokument, dass die Fluoreneinheit erforderlich ist, um besonders gute Ergebnisse in organischen Elektrolumineszenzvorrichtungen zu erzielen.

Weiterhin offenbaren die Dokumente JP 11-111460 A, JP 2000-007604 A, US 2003/0111692 A1, JP 2005-008587 A, US 2004/0263067 A1, EP 1182183 A1, EP 0681019 A2 und EP 1167488 A1 Bis-Anthracenderivate und deren Verwendung in OLEDs.

Überraschend wurde gefunden, dass bestimmte, unten beschriebene, Anthracenderivate deutliche Verbesserungen gegenüber dem oben beschriebenen Stand der Technik aufweisen. Mit diesen Verbindungen können höhere Effizienzen und verbesserte Lebensdauern erhalten werden. Außerdem weisen diese Verbindungen eine geringere Kristallisationsneigung und eine höhere Glasübergangstemperatur auf als Verbindungen gemäß dem Stand der Technik. Diese Verbindungen und deren Verwendung in OLEDs sind daher der Gegenstand der vorliegenden Erfindung.

Gegenstand der Erfindung sind Verbindungen gemäß Anspruch 1.

Unter einer cyclischen Alkylgruppe im Sinne dieser Erfindung werden sowohl monocyclische wie auch bi- und polycyclische Alkylgruppen verstanden.

Unter benachbarten Substituenten im Sinne dieser Erfindung werden Substituenten verstanden, die an direkt benachbarten C-Atomen gebunden sind, also an C-Atomen, welche eine direkte Bindung aufweisen.

Unter einer Arylgruppe bzw. einer Heteroarylgruppe im Sinne dieser Erfindung wird eine aromatische Gruppe bzw. heteroaromatische Gruppe mit einem gemeinsamen aromatischen π-Elektronensystem verstanden. Dies kann im Sinne dieser Erfindung ein einfacher Homo- oder Heterocyclus sein, beispielsweise Benzol, Pyridin, Thiophen, etc., oder es kann ein kondensiertes aromatisches Ringsystem sein, in dem mindestens zwei aromatische oder heteroaromatische Ringe, beispielsweise Benzolringe, miteinander "verschmolzen", d. h. durch Anellierung einander ankondensiert sind, also mindestens eine gemeinsame Kante und dadurch auch ein gemeinsames aromatisches π-Elektronensystem aufweisen. Diese Aryl- oder Heteroarylgruppen können substituiert oder unsubstituiert sein. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen darstellen, da es sich hierbei um separate aromatische Elektronensysteme handelt.

Ein aromatisches Ringsystem im Sinne dieser Erfindung enthält 6 bis 30 C-Atome im Ringsystem. Ein heteroaromatisches Ringsystem im Sinne dieser Erfindung enthält 2 bis 30 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (weniger als 10 % der von H verschiedenen Atome, bevorzugt weniger als 5 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, Fluoren, Triarylamin, Diarylether, etc. als aromatische Ringsysteme im Sinne dieser Erfindung verstanden werden. Dabei kann ein Teil des aromatischen oder heteroaromatischen Ringsystems auch eine kondensierte Gruppe sein.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 1 bis 30 aromatischen Ringatomen, welches noch jeweils mit den oben genannten Resten R¹ bzw. R² substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Tetracen, Pentacen, Benzpyren, Biphenyl, Biphenylen, Terphenyl, Terphenylen, Fluoren, Spirobifluoren, Truxen, Isotruxen, Dihydrophenanthren, Dihydropyren, Tetrahydropyren, cis- oder trans-Indenofluoren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausführungsform der Verbindung gemäß Formel (3) stehen maximal zwei Symbole X für N und die anderen Symbole X für CR¹. Bevorzugte Gruppen Ar², bzw. zentrale Einheiten in der Verbindung gemäß Formel (3), sind die Gruppen der folgenden Formeln (5) bis (10). Besonders bevorzugt sind darunter die Gruppen der Formeln (5) und (9), ganz besonders bevorzugt ist die Gruppe der Formel (5).

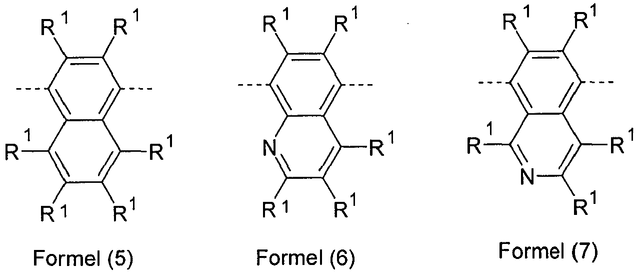

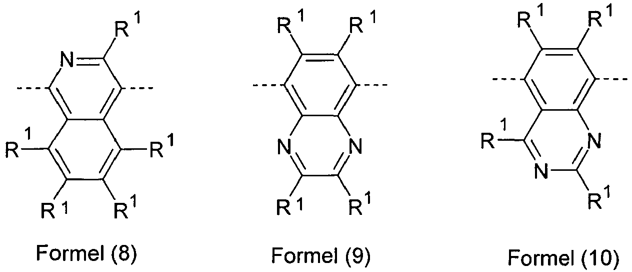

Besonders bevorzugt sind weiterhin Verbindungen der Formel (3), in denen das Symbol Ar¹, gleich oder verschieden bei jedem Auftreten, für eine 1-Naphthyl-, 2-Naphthyl-, 9-Anthryl-, 2-Phenanthrenyl-, 9-Phenanthrenyl-, Chinolinyl-, Isochinolinyl-, Dibenzothienyl-, Dibenzofuranyl-, Carbazolyl-, welches jeweils über C oder N verknüpft sein kann, Chinolinyl-, Isochinolinyl-, ortho-Biphenyl- oder 2-Fluorenylgruppe steht, die jeweils mit einem oder mehreren Resten R¹ substituiert sein kann, insbesondere für 1-Naphthyl, 2-Naphthyl, 9-Phenanthrenyl, Carbazolyl, Chinolinyl, ortho-Biphenyl oder 2-Fluorenyl, welches jeweils mit einem oder mehreren Resten R¹ substituiert sein kann. Bevorzugt sind die beiden Gruppen Ar¹ identisch.

Bevorzugt sind weiterhin Verbindungen gemäß Formel (3), in denen das Symbol R¹, gleich oder verschieden bei jedem Auftreten, für H, F, eine geradkettige Alkyl- oder Alkoxygruppe mit 1 bis 6 C-Atomen oder eine verzweigte oder cyclische Alkyl- oder Alkoxygruppe mit 3 bis 10 C-Atomen, wobei jeweils ein oder mehrere CH₂-Gruppen durch -O-, -S- oder -N(R²)-ersetzt sein können und wobei jeweils ein oder mehrere H-Atome durch F ersetzt sein können, oder eine Kombination aus zwei oder drei dieser Systeme steht. Besonders bevorzugte Reste R¹ sind ausgewählt aus der Gruppe bestehend aus H, F, geradkettigen Alkylgruppen mit 1 bis 4 C-Atomen, verzweigten Alkylgruppen mit 3 bis 5 C-Atomen oder cyclischen Alkylgruppen mit 5 bis 10 C-Atomen, wobei jeweils ein oder mehrere H-Atome durch F ersetzt sein können, oder eine Kombination aus zwei dieser Systeme.

Bevorzugt sind weiterhin Verbindungen gemäß Formel (3), in denen der Index n für 0, 1 oder 2 steht, besonders bevorzugt für 0 oder 1, ganz besonders bevorzugt für 0. Falls der Index n für 1 steht, ist der Substituent R¹ bevorzugt in 2-Position und/oder in 6-Position des Anthracens gebunden.

Bevorzugt sind weiterhin Verbindungen gemäß Formel (3), in denen der Index m für 1, 2 oder 3 steht, besonders bevorzugt für 1 oder 2.

Bevorzugt sind weiterhin Verbindungen gemäß Formel (3), deren Molekulargewicht zwischen 600 und 2000 g/mol liegt, besonders bevorzugt zwischen 700 und 1500 g/mol.

Dabei sei betont, dass sowohl Verbindungen gemäß Formel (3) erfindungsgemäß sind, in denen die beiden Gruppen Ar¹ gleich gewählt sind, wie auch Verbindungen, in denen die beiden Gruppen Ar¹ unterschiedlich sind. Bevorzugt sind Verbindungen, in denen die beiden Gruppen Ar¹ gleich sind und auch gleich substituiert sind.

Je nach Wahl von Ar¹ weisen die Verbindungen gemäß Formel (3) gehinderte Rotation um eine oder mehrere Bindungen (Bindungen zwischen Ar¹ und Anthracen) auf und sind dadurch zur Bildung von Atropisomeren befähigt, also zur Bildung von Stereoisomeren, welche bei Raumtemperatur stabil sind. Wenn die Verbindung gemäß Formel (3) Atropisomerie um eine oder um mehrere Bindungen zeigt, so sind jeweils auch die entsprechenden angereicherten oder isolierten Atropisomere Gegenstand der Erfindung. Dies bezieht sich sowohl auf Enantiomere wie auch auf Diastereomere. Durch die Wahl geeigneter Atropisomere lassen sich beispielsweise die Löslichkeit der Verbindung, die Glasübergangstemperatur und die elektrooptischen Eigenschaften beeinflussen. Bevorzugt sind Verbindungen, die keine Atropisomerie aufweisen.

Beispiele für bevorzugte Verbindungen gemäß Formel (3) sind die in der folgenden Tabelle 1 aufgeführten Verbindungen. Ph steht für eine Phenylgruppe. Die in die Tabelle eingetragenen Abkürzungen stehen für die folgenden Gruppen, wobei die gestrichelte Linie jeweils die Verknüpfung mit dem Anthracen andeutet:
Ar¹ : 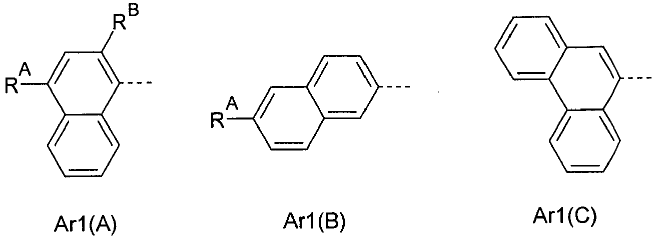 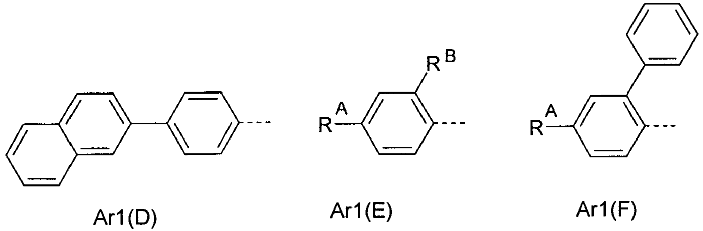 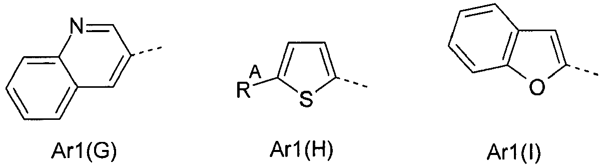
Ar²:

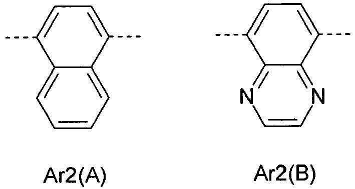

**Tabelle 1 (Verbindungen mit Markierung # sind nicht anspruchsgemäß):**

| Nr. | Ar¹ | Ar² | m | Ar¹ | |
|---|---|---|---|---|---|
| | | | | R^{A} | R^{B} |
| 1 | Ar1(A) | Ar2(A) | 1 | H | H |
| 2 | Ar1(A) | Ar2(A) | 2 | H | H |
| 3 | Ar1(A) | Ar2(B) | 1 | H | H |
| 4 | Ar1(A) | Ar2(B) | 2 | H | H |
| 5 | Ar1(A) | Ar2(A) | 1 | H | F |
| 6 | Ar1(A) | Ar2(A) | 2 | H | F |
| 7 | Ar1(A) | Ar2(B) | 1 | H | F |
| 8 | Ar1(A) | Ar2(B) | 2 | H | F |
| 9 | Ar1(A) | Ar2(A) | 1 | H | CH3 |
| 10 | Ar1(A) | Ar2(A) | 2 | H | CH3 |
| 11 | Ar1(A) | Ar2(B) | 1 | H | CH3 |
| 12 | Ar1(A) | Ar2(B) | 2 | H | CH3 |
| 13 | Ar1(A) | Ar2(A) | 1 | H | Ph |
| 14 | Ar1(A) | Ar2(A) | 2 | H | Ph |
| 15 | Ar1(A) | Ar2(B) | 1 | H | Ph |
| 16 | Ar1(A) | Ar2(B) | 2 | H | Ph |
| 17 | Ar1(A) | Ar2(A) | 1 | F | H |
| 18 | Ar1(A) | Ar2(A) | 2 | F | H |
| 19 | Ar1(A) | Ar2(B) | 1 | F | H |
| 20 | Ar1(A) | Ar2(B) | 2 | F | H |
| 21 | Ar1(A) | Ar2(A) | 1 | F | F |
| 22 | Ar1(A) | Ar2(A) | 2 | F | F |
| 23 | Ar1(A) | Ar2(B) | 1 | F | F |
| 24 | Ar1(A) | Ar2(B) | 2 | F | F |
| 25 | Ar1(A) | Ar2(A) | 1 | F | CH3 |
| 26 | Ar1(A) | Ar2(A) | 2 | F | CH3 |
| 27 | Ar1(A) | Ar2(B) | 1 | F | CH3 |
| 28 | Ar1(A) | Ar2(B) | 2 | F | CH3 |
| 29 | Ar1(A) | Ar2(A) | 1 | F | Ph |
| 30 | Ar1(A) | Ar2(A) | 2 | F | Ph |
| 31 | Ar1(A) | Ar2(B) | 1 | F | Ph |
| 32 | Ar1(A) | Ar2(B) | 2 | F | Ph |
| 33 | Ar1(A) | Ar2(A) | 1 | CH3 | H |
| 34 | Ar1(A) | Ar2(A) | 2 | CH3 | H |
| 35 | Ar1(A) | Ar2(B) | 1 | CH3 | H |
| 36 | Ar1(A) | Ar2(B) | 2 | CH3 | H |
| 37 | Ar1(A) | Ar2(A) | 1 | CH3 | F |
| 38 | Ar1(A) | Ar2(A) | 2 | CH3 | F |
| 39 | Ar1(A) | Ar2(B) | 1 | CH3 | F |
| 40 | Ar1(A) | Ar2(B) | 2 | CH3 | F |
| 41 | Ar1(A) | Ar2(A) | 1 | CH3 | CH3 |
| 42 | Ar1(A) | Ar2(A) | 2 | CH3 | CH3 |
| 43 | Ar1(A) | Ar2(B) | 1 | CH3 | CH3 |
| 44 | Ar1(A) | Ar2(B) | 2 | CH3 | CH3 |
| 45 | Ar1(A) | Ar2(A) | 1 | CH3 | Ph |
| 46 | Ar1(A) | Ar2(A) | 2 | CH3 | Ph |
| 47 | Ar1(A) | Ar2(B) | 1 | CH3 | Ph |
| 48 | Ar1(A) | Ar2(B) | 2 | CH3 | Ph |
| 49 | Ar1(A) | Ar2(A) | 1 | Ph | H |
| 50 | Ar1(A) | Ar2(A) | 2 | Ph | H |
| 51 | Ar1(A) | Ar2(B) | 1 | Ph | H |
| 52 | Ar1(A) | Ar2(B) | 2 | Ph | H |
| 53 | Ar1(A) | Ar2(A) | 1 | Ph | F |
| 54 | Ar1(A) | Ar2(A) | 2 | Ph | F |
| 55 | Ar1(A) | Ar2(B) | 1 | Ph | F |
| 56 | Ar1(A) | Ar2(B) | 2 | Ph | F |
| 57 | Ar1(A) | Ar2(A) | 1 | Ph | CH3 |
| 58 | Ar1(A) | Ar2(A) | 2 | Ph | CH3 |
| 59 | Ar1(A) | Ar2(B) | 1 | Ph | CH3 |
| 60 | Ar1(A) | Ar2(B) | 2 | Ph | CH3 |
| 61 | Ar1(A) | Ar2(A) | 1 | Ph | Ph |
| 62 | Ar1(A) | Ar2(A) | 2 | Ph | Ph |
| 63 | Ar1(A) | Ar2(B) | 1 | Ph | Ph |
| 64 | Ar1(A) | Ar2(B) | 2 | Ph | Ph |
| 65 | Ar1(B) | Ar2(A) | 1 | H | - |
| 66 | Ar1(B) | Ar2(A) | 2 | H | - |
| 67 | Ar1(B) | Ar2(B) | 1 | H | - |
| 68 | Ar1(B) | Ar2(B) | 2 | H | - |
| 69 | Ar1(B) | Ar2(A) | 1 | F | - |
| 70 | Ar1(B) | Ar2(A) | 2 | F | - |
| 71 | Ar1(B) | Ar2(B) | 1 | F | - |
| 72 | Ar1(B) | Ar2(B) | 2 | F | - |
| 73 | Ar1(B) | Ar2(A) | 1 | CH3 | - |
| 74 | Ar1(B) | Ar2(A) | 2 | CH3 | - |
| 75 | Ar1(B) | Ar2(B) | 1 | CH3 | - |
| 76 | Ar1(B) | Ar2(B) | 2 | CH3 | - |
| 77 | Ar1(B) | Ar2(A) | 1 | Ph | - |
| 78 | Ar1(B) | Ar2(A) | 2 | Ph | - |
| 79 | Ar1(B) | Ar2(B) | 1 | Ph | - |
| 80 | Ar1(B) | Ar2(B) | 2 | Ph | - |
| 81 | Ar1(C) | Ar2(A) | 1 | - | - |
| 82 | Ar1(C) | Ar2(A) | 2 | - | - |
| 83 | Ar1(C) | Ar2(B) | 1 | - | - |
| 84 | Ar1(C) | Ar2(B) | 2 | - | - |
| 85 # | Ar1(D) | Ar2(A) | 1 | H | - |
| 86 # | Ar1(D) | Ar2(A) | 2 | H | - |
| 87 # | Ar1(D) | Ar2(B) | 1 | H | - |
| 88 # | Ar1(D) | Ar2(B) | 2 | H | - |
| 89 # | Ar1(D) | Ar2(A) | 1 | F | - |
| 90 # | Ar1(D) | Ar2(A) | 2 | F | - |
| 91 # | Ar1(D) | Ar2(B) | 1 | F | - |
| 92 # | Ar1(D) | Ar2(B) | 2 | F | - |
| 93 # | Ar1(D) | Ar2(A) | 1 | CH3 | - |
| 94 # | Ar1(D) | Ar2(A) | 2 | CH3 | - |
| 95 # | Ar1(D) | Ar2(B) | 1 | CH3 | - |
| 96 # | Ar1(D) | Ar2(B) | 2 | CH3 | - |
| 97 # | Ar1(D) | Ar2(A) | 1 | Ph | - |
| 98 # | Ar1(D) | Ar2(A) | 2 | Ph | - |
| 99 # | Ar1(D) | Ar2(B) | 1 | Ph | - |
| 100 # | Ar1(D) | Ar2(B) | 2 | Ph | - |
| 101 # | Ar1(E) | Ar2(A) | 1 | H | H |
| 102 # | Ar1(E) | Ar2(A) | 2 | H | H |
| 103 # | Ar1(E) | Ar2(B) | 1 | H | H |
| 104 # | Ar1(E) | Ar2(B) | 2 | H | H |
| 105 # | Ar1(E) | Ar2(A) | 1 | H | F |
| 106 # | Ar1(E) | Ar2(A) | 2 | H | F |
| 107 # | Ar1(E) | Ar2(B) | 1 | H | F |
| 108 # | Ar1(E) | Ar2(B) | 2 | H | F |
| 109 # | Ar1(E) | Ar2(A) | 1 | H | CH3 |
| 110 # | Ar1(E) | Ar2(A) | 2 | H | CH3 |
| 111 # | Ar1(E) | Ar2(B) | 1 | H | CH3 |
| 112 # | Ar1(E) | Ar2(B) | 2 | H | CH3 |
| 113 | Ar1(E) | Ar2(A) | 1 | H | Ph |
| 114 | Ar1(E) | Ar2(A) | 2 | H | Ph |
| 115 | Ar1(E) | Ar2(B) | 1 | H | Ph |
| 116 | Ar1(E) | Ar2(B) | 2 | H | Ph |
| 117 # | Ar1(E) | Ar2(A) | 1 | F | H |
| 118 # | Ar1(E) | Ar2(A) | 2 | F | H |
| 119 # | Ar1(E) | Ar2(B) | 1 | F | H |
| 120 # | Ar1(E) | Ar2(B) | 2 | F | H |
| 121 # | Ar1(E) | Ar2(A) | 1 | F | F |
| 122 # | Ar1(E) | Ar2(A) | 2 | F | F |
| 123 # | Ar1(E) | Ar2(B) | 1 | F | F |
| 124 # | Ar1(E) | Ar2(B) | 2 | F | F |
| 125 # | Ar1(E) | Ar2(A) | 1 | F | CH3 |
| 126 # | Ar1(E) | Ar2(A) | 2 | F | CH3 |
| 127 # | Ar1(E) | Ar2(B) | 1 | F | CH3 |
| 128 # | Ar1(E) | Ar2(B) | 2 | F | CH3 |
| 129 | Ar1(E) | Ar2(A) | 1 | F | Ph |
| 130 | Ar1(E) | Ar2(A) | 2 | F | Ph |
| 131 | Ar1(E) | Ar2(B) | 1 | F | Ph |
| 132 | Ar1(E) | Ar2(B) | 2 | F | Ph |
| 133 # | Ar1(E) | Ar2(A) | 1 | CH3 | H |
| 134 # | Ar1(E) | Ar2(A) | 2 | CH3 | H |
| 135 # | Ar1(E) | Ar2(B) | 1 | CH3 | H |
| 136 # | Ar1(E) | Ar2(B) | 2 | CH3 | H |
| 137 # | Ar1(E) | Ar2(A) | 1 | CH3 | F |
| 138 # | Ar1(E) | Ar2(A) | 2 | CH3 | F |
| 139 # | Ar1(E) | Ar2(B) | 1 | CH3 | F |
| 140 # | Ar1(E) | Ar2(B) | 2 | CH3 | F |
| 141 # | Ar1(E) | Ar2(A) | 1 | CH3 | CH3 |
| 142 # | Ar1(E) | Ar2(A) | 2 | CH3 | CH3 |
| 143 # | Ar1(E) | Ar2(B) | 1 | CH3 | CH3 |
| 144 # | Ar1(E) | Ar2(B) | 2 | CH3 | CH3 |
| 145 | Ar1(E) | Ar2(A) | 1 | CH3 | Ph |
| 146 | Ar1(E) | Ar2(A) | 2 | CH3 | Ph |
| 147 | Ar1(E) | Ar2(B) | 1 | CH3 | Ph |
| 148 | Ar1(E) | Ar2(B) | 2 | CH3 | Ph |
| 149 | Ar1(E) | Ar2(A) | 1 | Ph | H |
| 150 | Ar1(E) | Ar2(A) | 2 | Ph | H |
| 151 | Ar1(E) | Ar2(B) | 1 | Ph | H |
| 152 | Ar1(E) | Ar2(B) | 2 | Ph | H |
| 153 | Ar1(E) | Ar2(A) | 1 | Ph | F |
| 154 | Ar1(E) | Ar2(A) | 2 | Ph | F |
| 155 | Ar1(E) | Ar2(B) | 1 | Ph | F |
| 156 | Ar1(E) | Ar2(B) | 2 | Ph | F |
| 157 | Ar1(E) | Ar2(A) | 1 | Ph | CH3 |
| 158 | Ar1(E) | Ar2(A) | 2 | Ph | CH3 |
| 159 | Ar1(E) | Ar2(B) | 1 | Ph | CH3 |
| 160 | Ar1(E) | Ar2(B) | 2 | Ph | CH3 |
| 161 | Ar1(E) | Ar2(A) | 1 | Ph | Ph |
| 162 | Ar1(E) | Ar2(A) | 2 | Ph | Ph |
| 163 | Ar1(E) | Ar2(B) | 1 | Ph | Ph |
| 164 | Ar1(E) | Ar2(B) | 2 | Ph | Ph |
| 165 | Ar1(F) | Ar2(A) | 1 | H | - |
| 166 | Ar1(F) | Ar2(A) | 2 | H | - |
| 167 | Ar1(F) | Ar2(B) | 1 | H | - |
| 168 | Ar1(F) | Ar2(B) | 2 | H | - |
| 169 | Ar1(F) | Ar2(A) | 1 | F | - |
| 170 | Ar1(F) | Ar2(A) | 2 | F | - |
| 171 | Ar1(F) | Ar2(B) | 1 | F | - |
| 172 | Ar1(F) | Ar2(B) | 2 | F | - |
| 173 | Ar1(F) | Ar2(A) | 1 | CH3 | - |
| 174 | Ar1(F) | Ar2(A) | 2 | CH3 | - |
| 175 | Ar1(F) | Ar2(B) | 1 | CH3 | - |
| 176 | Ar1(F) | Ar2(B) | 2 | CH3 | - |
| 177 | Ar1(F) | Ar2(A) | 1 | Ph | - |
| 178 | Ar1(F) | Ar2(A) | 2 | Ph | - |
| 179 | Ar1(F) | Ar2(B) | 1 | Ph | - |
| 180 | Ar1(F) | Ar2(B) | 2 | Ph | - |
| 181 | Ar1(G) | Ar2(A) | 1 | - | - |
| 182 | Ar1(G) | Ar2(A) | 2 | - | - |
| 183 | Ar1(G) | Ar2(B) | 1 | - | - |
| 184 | Ar1(G) | Ar2(B) | 2 | - | - |
| 185 # | Ar1(H) | Ar2(A) | 1 | H | - |
| 186 # | Ar1(H) | Ar2(A) | 2 | H | - |
| 187 # | Ar1(H) | Ar2(B) | 1 | H | - |
| 188 # | Ar1(H) | Ar2(B) | 2 | H | - |
| 189 # | Ar1(H) | Ar2(A) | 1 | F | - |
| 190 # | Ar1(H) | Ar2(A) | 2 | F | - |
| 191 # | Ar1(H) | Ar2(B) | 1 | F | - |
| 192 # | Ar1(H) | Ar2(B) | 2 | F | - |
| 193 # | Ar1(H) | Ar2(A) | 1 | CH3 | - |
| 194 # | Ar1(H) | Ar2(A) | 2 | CH3 | - |
| 195 # | Ar1(H) | Ar2(B) | 1 | CH3 | - |
| 196 # | Ar1(H) | Ar2(B) | 2 | CH3 | - |
| 197 # | Ar1(H) | Ar2(A) | 1 | Ph | - |
| 198 # | Ar1(H) | Ar2(A) | 2 | Ph | - |
| 199 # | Ar1(H) | Ar2(B) | 1 | Ph | - |
| 200 # | Ar1(H) | Ar2(B) | 2 | Ph | - |
| 201 # | Ar1(I) | Ar2(A) | 1 | - | - |
| 202 # | Ar1(I) | Ar2(A) | 2 | - | - |
| 203 # | Ar1(I) | Ar2(B) | 1 | - | - |
| 204 # | Ar1(I) | Ar2(B) | 2 | - | - |

Die erfindungsgemäßen Verbindungen können durch eine Folge von Übergangsmetall-katalysierten Kupplungsreaktionen synthetisiert werden. Als Kupplungsreaktion hat sich insbesondere die Suzuki-Kupplung von Arylboronsäurederivaten, beispielsweise Arylboronsäuren oder Arylboronsäureestern, und aromatischen Halogeniden bewährt, insbesondere unter Palladium-Katalyse. Die typischen Reaktionsbedingungen für die Suzuki-Kupplung sind dem Fachmann bekannt. Ebenso ist dem Fachmann bekannt, dass sich als Halogenide insbesondere die Bromide und Iodide eignen, dass aber auch andere Abgangsgruppen, wie beispielsweise Tosylat, Triflat oder allgemein Sulfonate verwendet werden können. So kann beispielsweise ein Diboronsäurederivat der zentralen aromatischen Einheit synthetisiert werden, welches in einer Suzuki-Kupplung mit einem, gegebenenfalls substituierten, 9-Halogenanthracen gekuppelt wird. In einem weiteren Schritt kann das Anthracen in 10-Position halogeniert, beispielsweise mit NBS bromiert, werden. Die halogenierte Verbindung kann in einem weiteren Schritt mit einem Boronsäurederivat der Gruppe Ar¹ in einer Suzuki-Kupplung zur Verbindung gemäß Formel (3), gekuppelt werden. Ebenso kann umgekehrt zunächst ein Boronsäurederivat der Gruppe Ar¹ in einer Suzuki-Kupplung mit einem gegebenenfalls substituierten 9-Halogenanthracen gekuppelt werden, welches in einem weiteren Schritt in 10-Position, beispielsweise mit NBS, halogeniert werden kann. Die halogenierte Verbindung kann in einem weiteren Schritt mit einem Diboronsäurederivat der zentralen Einheit in einer Suzuki-Kupplung zur Verbindung gemäß Formel (3) gekuppelt werden oder kann, nach Umsetzung zu einem Boronsäurederivat, mit einem Dihalogenid der zentralen Einheit gekuppelt werden. Diese Verfahren sind unabhängig von der genauen Struktur der Gruppen Ar¹ und der zentralen Einheit und finden gleichermaßen für aromatische und heteroaromatische Gruppen als zentrale Einheit oder als Ar¹ Anwendung. Statt der Suzuki-Kupplung kommen auch weitere metallkatalysierte Kupplungsreaktionen in Frage, wie beispielsweise die Stille-Kupplung, also die Kupplung von Organozinnverbindungen unter Palladium-Katalyse.

Ein weiterer Gegenstand der Erfindung ist ein Verfahren zur Synthese von Verbindungen gemäß Formel (3) ,dadurch gekennzeichnet, dass die Bindungen zwischen dem Anthracen und den Gruppen Ar¹ durch Suzuki-Kupplung geknüpft werden.

Die Verbindung gemäß der folgenden Formel (11) ist eine wertvolle Zwischenstufe zur Synthese von Verbindungen gemäß Formel (3) nach dem oben aufgeführten Verfahren.

Ein weiterer Gegenstand der Erfindung ist daher eine Verbindung gemäß Formel (11), 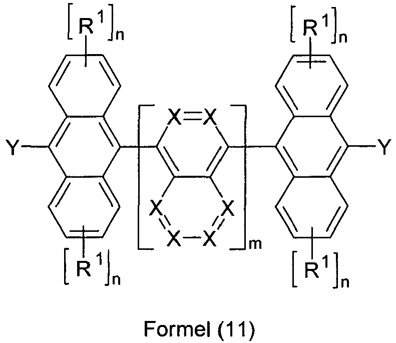 wobei Y, gleich oder verschieden bei jedem Auftreten, für Chlor, Brom, Iod oder eine Gruppe der Formel OSO₂R³ steht, wobei R³ eine organische Gruppe mit 1 bis 20 C-Atomen darstellt, in der auch einzelne H-Atome durch Fluor ersetzt sein können, insbesondere für Brom, und die weiteren Symbole und Indizes die oben aufgeführte Bedeutung haben.

Die Verbindungen gemäß Formel (3) eignen sich für den Einsatz in organischen elektronischen Vorrichtungen, insbesondere in organischen Elektrolumineszenzvorrichtungen.

Ein weiterer Gegenstand der Erfindung ist daher die Verwendung der Verbindungen gemäß Formel (3) in organischen elektronischen Vorrichtungen, insbesondere in organischen Elektrolumineszenzvorrichtungen.

Nochmals ein weiterer Gegenstand der Erfindung sind organische elektronische Vorrichtungen, enthaltend mindestens eine Verbindung gemäß Formel (3). Die organischen elektronischen Vorrichtungen sind bevorzugt ausgewählt aus organischen Elektrolumineszenzvorrichtungen (OLED, PLED), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), organischen Photorezeptoren oder organischen Laserdioden (O-Laser); besonders bevorzugt sind organische Elektrolumineszenzvorrichtungen (OLEDs, PLEDs).

Die organische Elektrolumineszenzvorrichtung enthält Anode, Kathode und mindestens eine emittierende Schicht und kann noch weitere Schichten enthalten. Diese können beispielsweise sein: Lochinjektionsschicht, Lochtransportschicht, Elektronentransportschicht, Elektroneninjektionsschicht und/oder eine Charge-Generation Layer (T. Matsumoto et al., Multiphoton Organic EL Device Having Charge Generation Layer, IDMC 2003, Taiwan; Session 21 OLED (5)). Die Materialien in diesen Schichten können auch dotiert sein. Es muss aber nicht notwendigerweise jede dieser Schichten vorhanden sein muss. Als Lochtransportmaterialien eignen sich beispielsweise aromatische Amine, wie sie üblicherweise gemäß dem Stand der Technik verwendet werden, welche auch p-dotiert sein können. Als Elektronentransportmaterialien eignen sich beispielsweise Metallchelatkomplexe, z. B. AlQ₃, Verbindungen auf Basis elektronenarmer Heterocyclen, z. B. Triazinderivate, Verbindungen enthaltend aromatische Carbonyle oder Phosphinoxide, wie z. B. beschrieben in WO 05/084081 und WO 05/084082, oder auch weitere Elektronentransportmaterialien gemäß dem Stand der Technik, welche jeweils auch n-dotiert sein können. Als Elektroneninjektionsmaterialien eignen sich insbesondere Fluoride und Oxide der Alkali- und Erdalkalimetalle, beispielsweise NaF, BaF₂, CaF₂, LiF oder Li₂O.

Die Verbindung gemäß Formel (3) wird bevorzugt in einer emittierenden Schicht eingesetzt. Dabei wird sie bevorzugt als Hostmaterial zusammen mit einem Dotanden eingesetzt. Unter einem Hostmaterial wird in einem System aus Host und Dotand (binäre Mischung) diejenige Komponente verstanden, die in dem System im höheren Anteil vorliegt. Bei einem System aus einem Host und mehreren Dotanden (ternäre und höhere Mischungen) wird als Host diejenige Komponente verstanden, deren Anteil der höchste in der Mischung ist. Dabei eignet sich die Verbindung gemäß Formel (3) insbesondere als Hostmaterial für blaue Singulettemitter, aber auch für grün oder rot emittierende Verbindungen.

Der Anteil der Verbindung gemäß Formel (3) in der Mischung beträgt zwischen 50.0 und 99.9 Gew.-%, bevorzugt zwischen 80.0 und 99.5 Gew.-%, besonders bevorzugt zwischen 90.0 und 99.0 Gew.-%. Der Anteil des bzw. der Dotanden in der Mischung beträgt entsprechend zwischen 0.1 und 50.0 Gew.-%, bevorzugt zwischen 0.5 und 20.0 Gew.-%, besonders bevorzugt zwischen 1.0 und 10.0 Gew.-%.

Bevorzugte Dotanden sind ausgewählt aus der Klasse der Monostyrylamine, der Distyrylamine, der Tristyrylamine, der Tetrastyrylamine, der Styrylphosphine, der Styrylether und der Arylamine. Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Phosphine und Ether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, bevorzugt mit mindestens 14 aromatischen Ringatomen. Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Besonders bevorzugte Dotanden sind gewählt aus den Klassen der Tristilbenamine, der aromatischen Stilbendiamine, der Anthracendiamine, der Pyrendiamine und der Chrysendiamine. Beispiele für derartige Dotanden sind substituierte oder unsubstituierte Tristilbenamine oder die Dotanden, die in WO 06/000388, WO 06/058737 und WO 06/000389 beschrieben sind.

Weiterhin bevorzugt sind organische Elektrolumineszenzvorrichtungen, dadurch gekennzeichnet, dass mehrere emittierende Schichten verwendet werden, wobei mindestens eine dieser Schichten mindestens eine Verbindung gemäß Formel (3) enthält. Besonders bevorzugt weisen diese Emissionsschichten insgesamt mehrere Emissionsmaxima zwischen 380 nm und 750 nm auf, so dass insgesamt weiße Emission resultiert, d. h. in der bzw. den weiteren emittierenden Schichten wird noch mindestens eine weitere emittierende Verbindung verwendet, die fluoreszieren oder phosphoreszieren kann. Insbesondere bevorzugt sind Dreischichtsysteme, wobei die drei Schichten blaue, grüne und orange oder rote Emission zeigen (für den prinzipiellen Aufbau siehe z. B. WO 05/011013).

Außer der Verbindung gemäß Formel (3) und dem bzw. den Dotanden können in der emittierenden Schicht auch weitere Substanzen vorhanden sein, beispielsweise Loch- oder Elektronentransportmaterialien.

In einer weiteren Ausführungsform der Erfindung werden die Verbindungen gemäß Formel (3) als emittierende Materialien eingesetzt, insbesondere als blau oder grün emittierende Materialien. Der Anteil der Verbindung gemäß Formel (3) in der Mischung der emittierenden Schicht beträgt dann zwischen 0.1 und 50.0 Gew.-%, bevorzugt zwischen 0.5 und 20.0 Gew.-%, besonders bevorzugt zwischen 1.0 und 10.0 Gew.-%. Entsprechend beträgt der Anteil des Hostmaterials zwischen 50.0 und 99.9 Gew.-%, bevorzugt zwischen 80.0 und 99.5 Gew.-%, besonders bevorzugt zwischen 90.0 und 99.0 Gew.-%.

Als Hostmaterialien kommen hierfür Materialien verschiedene Stoffklassen in Frage. Bevorzugte Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 und WO 05/084082), der Atropisomere (z. B. gemäß WO 06/048268) oder der Boronsäurederivate (z. B. gemäß WO 06/117052). Weiterhin kommen als Hostmaterialien auch die oben beschriebenen erfindungsgemäßen Verbindungen in Frage. Besonders bevorzugte Hostmaterialien sind außer den erfindungsgemäßen Verbindungen ausgewählt aus den Klassen der Oligoarylene, enthaltend Naphthalin, Anthracen und/oder Pyren oder Atropisomere dieser Verbindungen, der Oligoarylenvinylene, der Ketone, der Phosphinoxide und der Sulfoxide. Ganz besonders bevorzugte Hostmaterialien sind außer den erfindungsgemäßen Verbindungen ausgewählt aus den Klassen der Oligoarylene, enthaltend Anthracen und/oder Pyren oder Atropisomere dieser Verbindungen, der Phosphinoxide und der Sulfoxide. Unter einem Oligoarylen im Sinne dieser Erfindung soll eine Verbindung verstanden werden, in der mindestens drei Aryl- bzw. Arylengruppen aneinander gebunden sind.

In nochmals einer weiteren Ausführungsform der Erfindung werden die Verbindungen gemäß Formel (3), als Elektronentransportmaterial eingesetzt. Hier ist es bevorzugt, wenn eine oder mehrere Gruppen Ar¹ mindestens eine elektronenarme heterocyclische Verbindung enthalten, beispielsweise Stickstoffheterocyclen wie Pyridin, Pyrimidin, Chinolin etc. Weiterhin kann es bevorzugt sein, wenn die Verbindung mit Elektronendonor-Verbindungen dotiert ist.

Die Herstellung der organischen Elektrolumineszenzvorrichtung kann durch Aufbringen einer oder mehrerer Schichten durch ein Sublimationsverfahren erfolgen. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Die Herstellung kann weiterhin durch Aufbringen einer oder mehrerer Schichten mit dem OVPD (Organic Vapour Phase Deposition) Verfahren oder mit Hilfe einer Trägergassublimation erfolgen. Dabei werden die Materialien bei einem Druck zwischen 10⁻⁵ mbar und 1 bar aufgebracht.

Die Herstellung kann nochmals weiterhin durch Aufbringen einer oder mehrerer Schichten aus Lösung, wie z. B. durch Spincoating, oder mit einem beliebigen Druckverfahren, wie z. B. Siebdruck, Flexodruck oder Offsetdruck, bevorzugt aber LITI (Light Induced Thermal Imaging, Thermotransferdruck) oder Ink-Jet Druck (Tintenstrahldruck), erfolgen. Hierfür sind lösliche Verbindungen gemäß Formel (3), nötig. Hohe Löslichkeit lässt sich entweder durch geeignete Substitution der Verbindungen oder auch durch die Wahl geeigneter Atropisomere erreichen.

Ein weiterer Gegenstand der Erfindung ist daher ein Verfahren zur Herstellung organischer Elektrolumineszenzvorrichtungen, dadurch gekennzeichnet, dass mindestens eine Verbindung gemäß Formel (3), gegebenenfalls zusammen mit einem Dotanden und/oder anderen Verbindungen, durch ein Sublimationsverfahren oder aus Lösung, beispielsweise durch ein Druckverfahren, aufgebracht werden.

Die erfindungsgemäßen organischen Elektrolumineszenzvorrichtungen folgende überraschende Vorteile gegenüber dem Stand der Technik auf:
1. Die Stabilität der Vorrichtungen wird höher im Vergleich zu Systemen gemäß dem Stand der Technik, was sich vor allem in einer deutlich höheren Lebensdauer zeigt.
2. Die organischen Elektrolumineszenzvorrichtungen weisen eine deutlich höhere Effizienz auf, insbesondere bei blauer Lumineszenz, im Vergleich zu Systemen gemäß dem Stand der Technik.
3. Die erfindungsgemäßen Verbindungen weisen eine hohe Glasübergangstemperatur und eine geringe Kristallisationsneigung auf und eignen sich daher besonders zur Verwendung in organischen Elektrolumineszenzvorrichtungen.

Im vorliegenden Anmeldetext und auch in den im Weiteren folgenden Beispielen wird auf die Verwendung erfindungsgemäßer Mischungen in Bezug auf OLEDs und die entsprechenden Displays abgezielt. Trotz dieser Beschränkung der Beschreibung ist es für den Fachmann ohne erfinderisches Zutun möglich, die erfindungsgemäßen Verbindungen auch für die weiteren oben aufgeführten elektronischen Vorrichtungen zu verwenden.

Die Erfindung wird durch die nachfolgenden Beispiele näher erläutert, ohne sie dadurch einschränken zu wollen.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR bezogen bzw. nach literaturbekannten Synthesen dargestellt werden. Synthesebeispiele, die nicht anspruchsgemäß sind, sind mit # gekennzeichnet.

### Beispiel 1: Herstellung von 1,4-Bis-[9-(2-naphthyl)]-10-anthryl-naphthalin

### a) Naphthalin-1,4-diboronsäureethylenglycolester

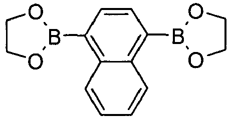

Eine Grignard-Lösung, hergestellt aus 223.5 g (780 mmol) Dibromnaphthalin und 41.5 g (1.6 mol) Magnesium in 1500 mL getrocknetem THF wird bei -75 °C zu 260 mL (2.34 mol) Trimethylborat in 500 mL THF getropft, 1 h bei -50 °C gerührt, danach auf RT erwärmt, mit 500 mL Wasser und 50 mL konz. Essigsäure hydrolysiert und extraktiv aufgearbeitet. Der nach Entfernen des Lösungsmittels erhaltene Rückstand wird mit 110 ml (1.6 mol) Ethylenglycol in 1000 mL Toluol am Wasserabscheider gekocht, das Lösungsmittel entfernt und der Rückstand aus Ethylacetat/Heptan umkristallisiert. Es verbleiben 157.1 g (75 %) Diester als hellgelber, kristalliner Feststoff.

### b) Herstellung von 1,4-Bis-(9-anthryl)-naphthalin

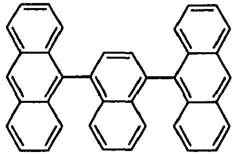

22.8 g (90 mmol) 9-Bromanthracen und 11.9 g (45 mmol) Naphthalin-1,4-diboronsäureethylenglycolester werden in 550 mL Dimethoxyethan und 140 mL Ethanol vorgelegt, 440 mL 2 M Na₂CO₃-Lösung zugegeben und das Gemisch mit Stickstoff gesättigt. Im Anschluss werden 3.2 g (10.7 mmol) Tris-*ortho*-tolylphosphin und 400 mg (1.8 mmol) Palladium(II)-acetat zugegeben und das Gemisch für 48 h zum Sieden erhitzt. Nach beendeter Reaktion werden 400 mL Wasser zugegeben, der Feststoff abgesaugt, mehrfach mit Wasser und EtOH gewaschen und im Vakuum getrocknet. Filtration und Umkristallisation aus 1,4-Dioxan liefert einen farblosen Feststoff (18.4 g, 87 %).

### c) Herstellung von 1,4-Bis-[(10-(9-bromanthryl)]-naphthalin

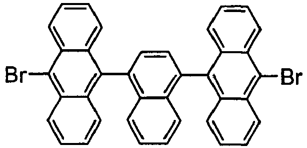

9.1 g (18.9 mmol) 1,4-Bis-(9-anthryl)-naphthalin werden in 150 mL Dichlormethan suspendiert, 7.1 g NBS zugegeben und die resultierende Suspension für 24 h bei RT gerührt. Das Reaktionsgemisch wird einrotiert, in EtOH/H₂O (1:1) ausgekocht und anschließend aus Dioxan umkristallisiert. Man erhält 11.2 g (93 %) eines fahlgelben Feststoffes mit einer Reinheit von > 98 % (RP-HPLC).

### d) Herstellung von 1,4-bis-[9-(2-Naphthyl)-10-anthryl]-naphthalin

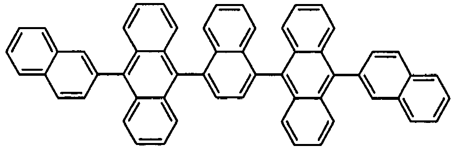

30.0 g (44.4 mmol) 1,4-Bis-[(10-(9-bromanthryl)]-naphthalin, 19.1 g (110.9 mmol) Naphthalin-2-boronsäure, 4.4 g (3.84 mmol) Palladiumtetrakis-triphenylphosphin und 440 mL 2 M Na₂CO₃-Lösung werden in 550 mL Dimethoxyethan und 140 mL Ethanol suspendiert und 48 h zum Sieden erhitzt. Der Feststoff wird abfiltriert, mit Wasser und Ethanol gewaschen, in Chloroform gelöst und filtriert. Anschließende Umkristallisation aus Toluol und Sublimation liefert einen fahlgelben Feststoff, der eine Reinheit von > 99.9 % aufweist (bestimmt durch RP-HPLC). Ausbeute: 20 g (68 %). T_{G} = 195 °C.

### Beispiele 2 - 14:

Analog zu Beispiel 1 werden folgende Verbindungen ausgehend von den aufgeführten Boronsäuren durch Kupplung nach dem in 1d) beschriebenen Verfahren mit dem in 1c) dargestellten Dibromid in den angegebenen Ausbeuten bei einer Reinheit von 99.9 % nach RP-HPLC dargestellt.

| **Bsp.** | **Boronsäure Kupplung analog 1c)** | **Produkt** | **Ausbeute** |
|---|---|---|---|
| 2 | 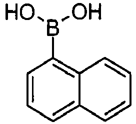 | 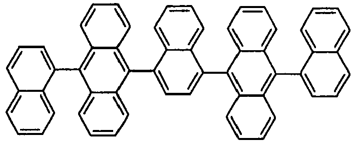 | 78%* |
| 3 | 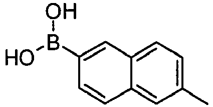 | 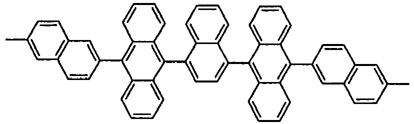 | 73% |
| 4 | 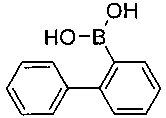 | 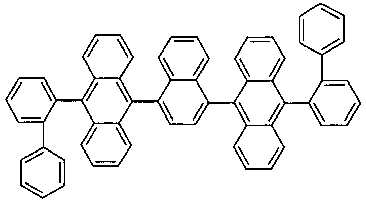 | 55%* |
| 5 | 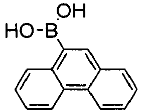 | 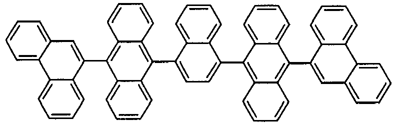 | 81%* |
| 6 # | 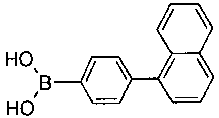 | 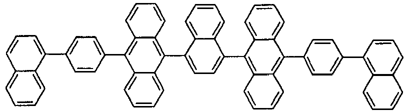 | 66% |
| 7 # | 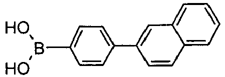 | 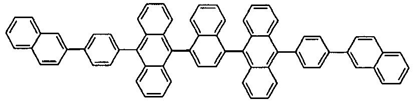 | 60% |
| 8 # | 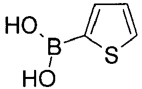 | 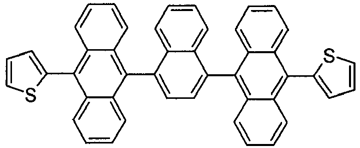 | 79% |
| 9 # | 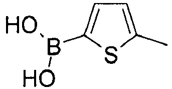 | 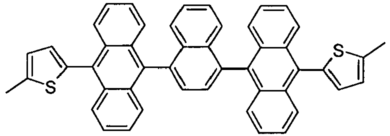 | 65% |
| 10 # | 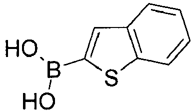 | 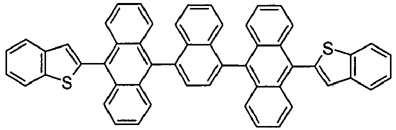 | 71% |
| 11 # | 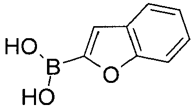 | 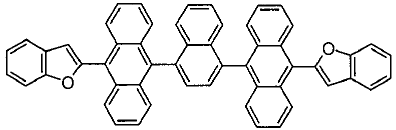 | 68% |
| 12 # | 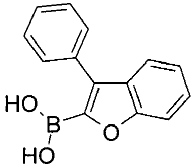 | 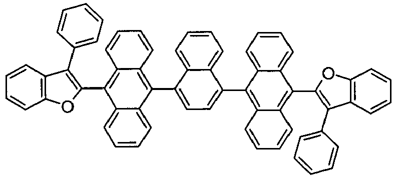 | 47% |
| 13 # | 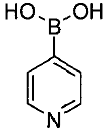 | 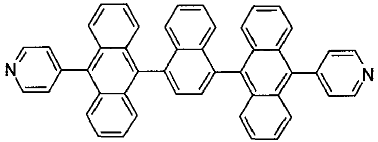 | 43% |
| 14 | 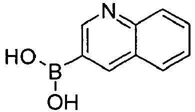 | 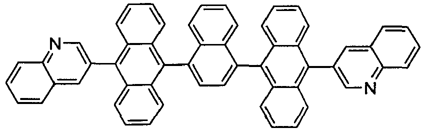 | 58% |

| | | | |
|---|---|---|---|
| *: Atropisomerengemisch, Reinheit einschließlich aller Isomere | | | |

### Beispiel 15: Herstellung von 4,4'-Bis-[9-(2-naphthyl)]-10-anthryl-1,1'-dinaphthalin

### a) Herstellung von 4,4'-Bis-(9-anthryl)-1,1'-dinaphthalin

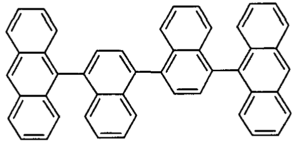

22.8 g (90 mmol) 9-Bromanthracen und 17.7 g (45 mmol) 1,1'-Binaphthyl-4,4'-diboronsäureethylenglycolester werden in 500 mL Toluol und 100 mL Dioxan vorgelegt, 500 mL 2 M Na₂CO₃-Lösung zugegeben und das Gemisch mit Stickstoff gesättigt. Im Anschluss werden 3.2 g (10.7 mmol) Tris-*ortho*-tolylphosphin und 400 mg (1.8 mmol) Palladium(II)acetat zugegeben und das Gemisch für 12 h zum Sieden erhitzt. Nach beendeter Reaktion werden 500 mL Wasser zugegeben, der Feststoff abgesaugt, mehrfach mit Wasser und EtOH gewaschen und im Vakuum getrocknet. Filtration und Umkristallisation aus 1,4-Dioxan liefert einen farblosen Feststoff (42.6 g, 78 %).

### b) Herstellung von 4,4'-Bis-[(10-(9-bromanthryl)]-1,1'-dinaphthalin

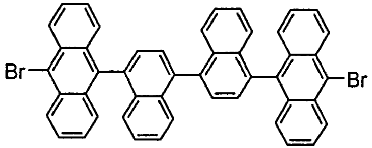

19.7 g (50 mmol) 4,4'-Bis-(9-anthryl)-1,1'-dinaphthalin werden in 500 mL Dichlormethan suspendiert, 18.7 g (105 mmol) NBS zugegeben und die resultierende Suspension für 18 h bei RT gerührt. Das Reaktionsgemisch wird einrotiert, in 500 ml EtOH/H₂O (1:1) ausgekocht und anschließend aus Dioxan umkristallisiert. Man erhält 36.7 g (96 %) eines fahlgelben Feststoffes mit einer Reinheit von > 98 % (RP-HPLC).

### c) Herstellung von 4,4'-Bis-[9-(2-Naphthyl)-10-anthryl]-1,1'-dinaphthalin

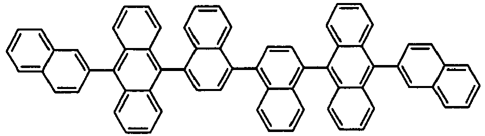

30.6 g (40 mmol) 1,4-Bis-[(10-(9-bromanthryl)]-naphthalin und 17.2 g (100 mmol) Naphthalin-2-boronsäure werden in 500 mL Toluol und 100 mL Dioxan vorgelegt, 500 mL 2 M Na₂CO₃-Lösung zugegeben und das Gemisch mit Stickstoff gesättigt. Im Anschluss werden 3.2 g (10.7 mmol) Tris-*ortho*-tolylphosphin und 400 mg (1.8 mmol) Palladium(II)-acetat zugegeben und das Gemisch für 12 h zum Sieden erhitzt. Der Feststoff wird abfiltriert, mit Wasser und Ethanol gewaschen, in Chloroform gelöst und filtriert. Anschließende Umkristallisation aus Toluol und Sublimation liefert einen fahlgelben Feststoff, der eine Reinheit von > 99.9 % aufweist (bestimmt durch RP-HPLC). Ausbeute: 24.4 g (71 %). T_{G} = 244 °C.

### Beispiele 16 - 28:

Analog zu Beispiel 15 werden folgende Verbindungen ausgehend von den aufgeführten Boronsäuren durch Kupplung nach dem in 15c) beschriebenen Verfahren mit dem in 15b) dargestellten Dibromid in den angegebenen Ausbeuten bei einer Reinheit von 99.9 % nach RP-HPLC dargestellt.

| **Bsp.** | **Boronsäure Kupplung analog 15c)** | **Produkt** | **Ausbeute** |
|---|---|---|---|
| 16 | 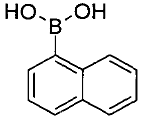 | 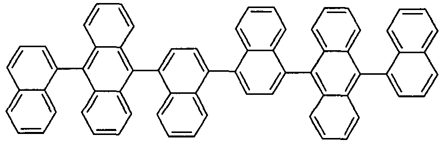 | 68%* |
| 17 | 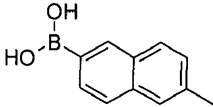 | 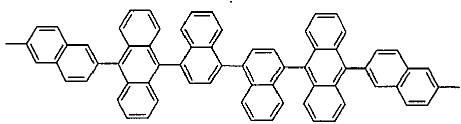 | 80% |
| 18 | 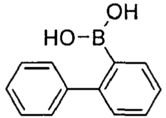 | 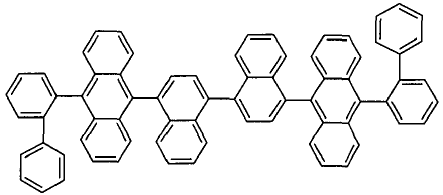 | 54%* |
| 19 | 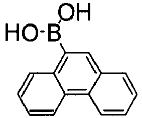 | 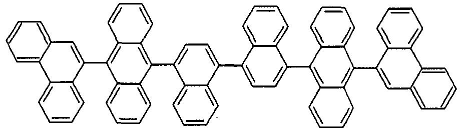 | 79%* |
| 20 # | 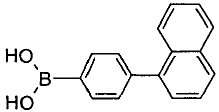 | 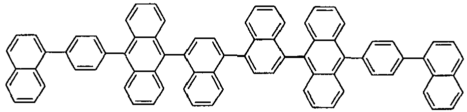 | 77% |
| 21 # | 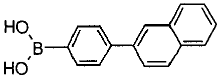 | 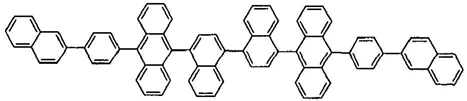 | 76% |
| 22 # | 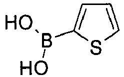 | 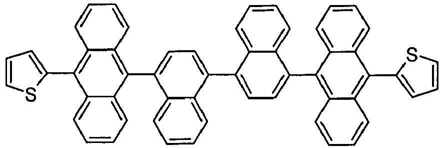 | 81% |
| 23 # | 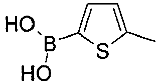 | 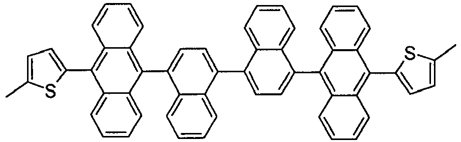 | 60% |
| 24 # | 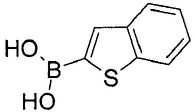 | 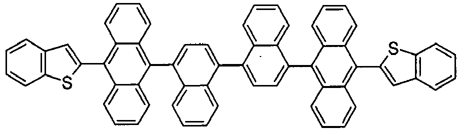 | 75% |
| 25 # | 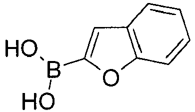 | 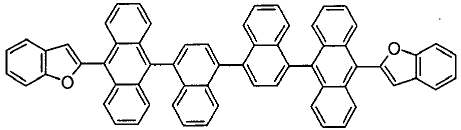 | 63% |
| 26 # | 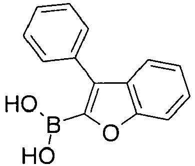 | 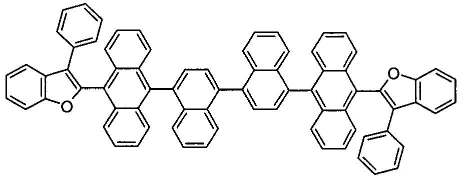 | 43% |
| 27 # | 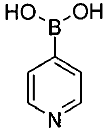 | 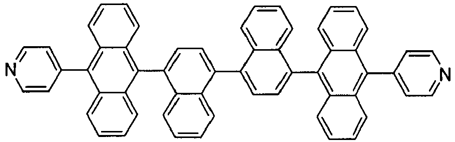 | 51% |
| 28 | 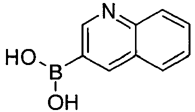 | 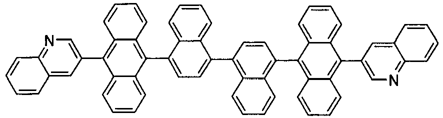 | 74% |

| | | | |
|---|---|---|---|
| *: Atropisomerengemisch, Reinheit einschließlich aller Isomere | | | |

### Beispiele 29 - 40:

Analog zu Beispiel 15 werden folgende Verbindungen ausgehend von den aufgeführten Boronsäuren durch Kupplung nach dem in 15a) beschriebenen Verfahren, anschließender Bromierung nach dem in 15b) beschriebenen Verfahren und anschließender erneuter Kupplung mit Chinolin-3-, Pyridin-4-, Benzothiophen-2- bzw. Benzofuran-2-boronsäure nach dem in 15c) beschriebenen Verfahren in den angegebenen Ausbeuten bei einer Reinheit von 99.9 % nach RP-HPLC dargestellt.

| **Bsp.** | **Boronsäure Kupplung analog 15a)** | **Produkt** | **Ausbeute** |
|---|---|---|---|
| 29 # | 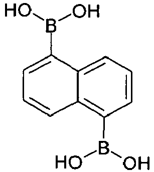 | 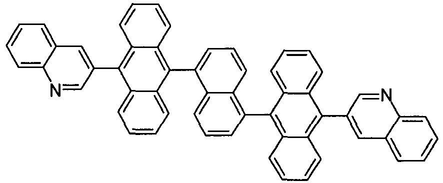 | 70% |
| 30 # | 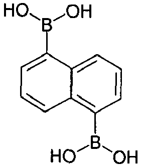 | 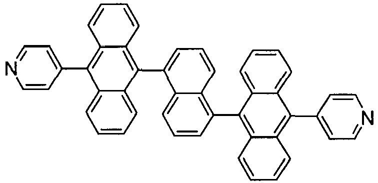 | 69% |
| 31 # | 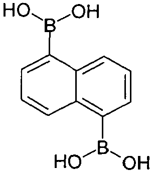 | 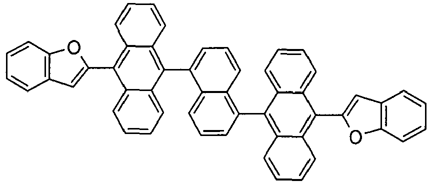 | 48% |
| 32 # | 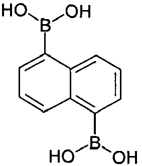 | 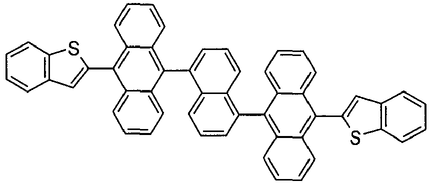 | 52% |
| 33 # | 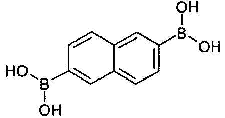 | 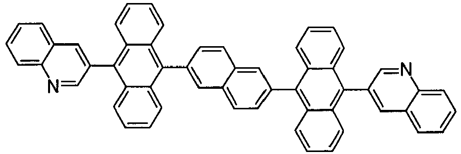 | 64% |
| 34 # | 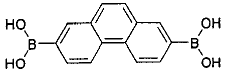 | 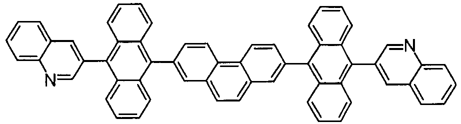 | 83% |
| 35 # | 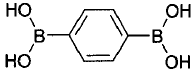 | 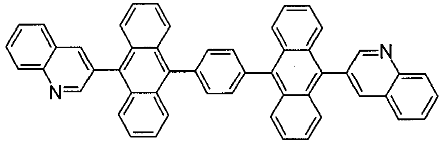 | 78% |
| 36 # | 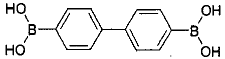 | 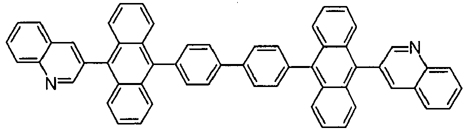 | 65% |
| 37 # | 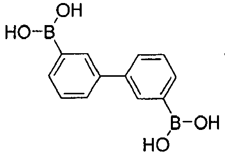 | 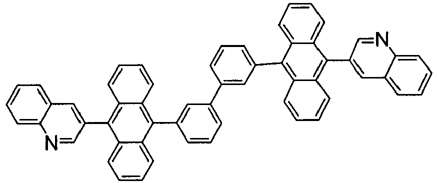 | 66% |
| 38 # | 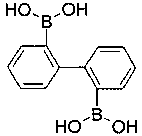 | 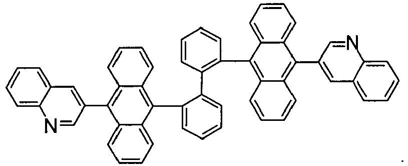 | 54% |
| 39 # | 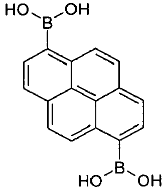 | 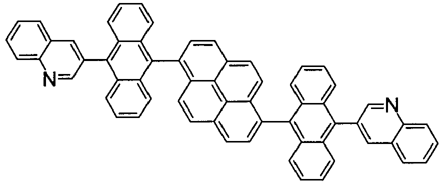 | 43% |
| 40 | 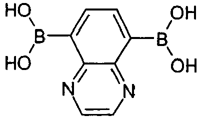 | 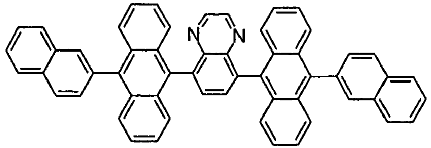 | 23% |

### Beispiel 41: Herstellung der OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 42 bis 63 werden die Ergebnisse verschiedener OLEDs vorgestellt. Glasplatten, die mit strukturiertem ITO (Indium-Zinn-Oxid) beschichtet sind, bilden die Substrate der OLEDs. Zur verbesserten Prozessierung wird PEDOT (aus Wasser aufgeschleudert; bezogen von H. C. Starck, Goslar, Deutschland; Poly(3,4-ethylendioxy-2,5-thiophen)) auf das Substrat aufgebracht. Die OLEDs bestehen aus folgender Schichtenfolge: Substrat / PEDOT 20 nm / Lochinjektionsschicht (HIL) aus dem Lochinjektionsmaterial HIL1 20nm / Lochtransportschicht (HTM) 20 nm / Emissionsschicht (EML) 30 nm / Elektronentransportschicht (ETM) 20 nm und abschließend eine Kathode. Die Materialien bis auf PEDOT werden in einer Vakuumkammer thermisch aufgedampft. Dabei besteht die Emissionsschicht immer aus einem Matrixmaterial (Host) und einem Dotierstoff (Dotand), der durch Coverdampfung dem Host beigemischt wird. Die Kathode wird durch eine 1 nm dünne LiF-Schicht und einer darauf abgeschiedenen 150 nm Al-Schicht gebildet. Die Tabelle 3 zeigt die chemischen Strukturen der zum Aufbau der OLEDs verwendeten Materialien.

Diese OLEDs werden standardmäßig charakterisiert; hierfür werden die Elektrolumineszenzspektren, die Effizienz (gemessen in cd/A), die Leistungseffizienz (gemessen in lm/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 2000 cd/m² auf die Hälfte gesunken ist. In Tabelle 4 sind die Ergebnisse einiger OLEDs (Beispiele 42 bis 63) zusammengefasst. Als erfindungsgemäße Hostmaterialien wurden die Verbindungen der Beispiele 1, 14, 16, 19 und 40 verwendet. Als Vergleichsbeispiele werden die Hosts H1 und H2 gemäß dem Stand der Technik verwendet. Verbindungen 6, 10, 35 und 37 werden in Referenzbeispielen verwendet.

**Tabelle 3**

| | | |
|---|---|---|
| 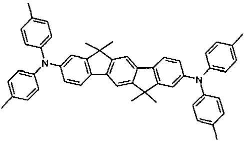 | 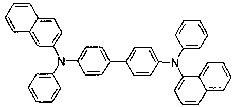 | 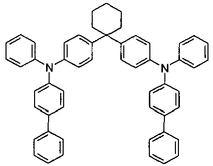 |
| HIL1 | HTM1 | HTM2 |
| 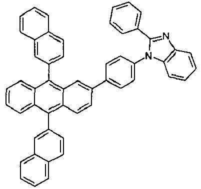 | 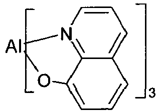 | 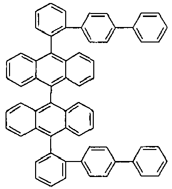 |
| ETM1 | ETM2 | Host H1 |
| 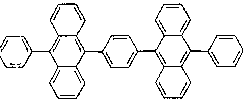 | 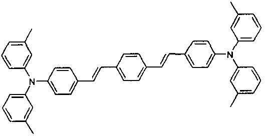 | 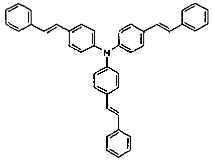 |
| Host H2 | Dotand D1 | Dotand D2 |
| 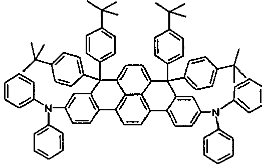 | 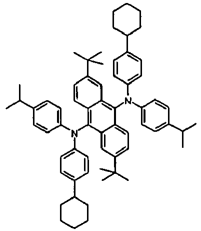 | |
| Dotand D3 | Dotand D4 | |

**Tabelle 4 (mit # markierte Beispiele sind nicht anspruchsgemäß)**

| **Beispiel** | **HTM** | **EML** | **ETM** | **Max. Effizienz (cd/A)** | **Spannung(V) bei 1000 cd/m²** | **CIE** | **Lebensdauer bei 2000 cd/m² (h)** |
|---|---|---|---|---|---|---|---|
| 42 (Vergleich) | HTM1 | H1 + 5% D1 | ETM1 | 10.9 | 5.8 | x=0.17 / y=0.33 | 3200 |
| 43 (Vergleich) | HTM1 | H2 + 5% D1 | ETM1 | 11.2 | 5.7 | x=0.17 / y=0.33 | 2500 |
| 44 (Vergleich) | HTM1 | H1 + 5% D2 | ETM1 | 3.6 | 6.3 | x=0.15 / y=0.13 | 700 |
| 45 (Vergleich) | HTM2 | H2 + 5% D2 | ETM1 | 3.2 | 6.5 | x=0.15 / y=0.17 | 300 |
| 46 (Vergleich) | HTM2 | H1 + 5% D3 | ETM2 | 20.4 | 5.4 | x=0.31 / y=0.63 | 7300 |
| 47 (Vergleich) | HTM2 | H1 + 5% D4 | ETM2 | 19.7 | 5.3 | x=0.28 / y=0.60 | 8500 |
| 48 | HTM1 | Bsp. 1 + 5% D1 | ETM1 | 12.3 | 5.5 | x=0.17 / y=0.33 | 5100 |
| 49 # | HTM1 | Bsp. 6 + 5% D1 | ETM1 | 13.0 | 5.7 | x=0.17 / y=0.33 | 5800 |
| 50 | HTM1 | Bsp. 16 + 5% D1 | ETM1 | 11.0 | 5.1 | x=0.17 / y=0.33 | 4900 |
| 51 | HTM1 | Bsp. 1 + 5% D2 | ETM1 | 4.1 | 5.5 | x=0.15 / y=0.15 | 2100 |
| 52 | HTM1 | Bsp. 6 + 5% D2 | ETM1 | 4.2 | 5.7 | x=0.15 / y=0.14 | 2500 |
| 53 | HTM1 | Bsp. 19 + 5% D2 | ETM1 | 4.5 | 5.6 | x=0.15 / y=0.12 | 2800 |
| 54 # | HTM2 | Bsp. 10 + 5% D3 | ETM2 | 24.4 | 5.8 | x=0.30 / y=0.63 | 7000 |
| 55 | HTM2 | Bsp. 14 + 5% D3 | ETM2 | 21.6 | 5.5 | x=0.30 / y=0.63 | 8100 |
| 56 | HTM2 | Bsp. 16 + 5% D3 | ETM2 | 21.3 | 5.6 | x=0.31 / y=0.63 | 8800 |
| 57 # | HTM2 | Bsp. 35 + 5% D4 | ETM2 | 23.7 | 5.1 | x=0.28 / y=0.60 | 9000 |
| 58 # | HTM2 | Bsp. 37 + 5% D4 | ETM2 | 26.1 | 5.2 | x=0.29 / y=0.59 | 8700 |
| 59 | HTM2 | Bsp. 40 + 5% D4 | ETM2 | 19.7 | 5.3 | x=0.28 / y=0.59 | 9300 |
| 60 | HTM2 | Bsp. 6 + 5% D4 | ETM2 | 24.3 | 5.0 | x=0.31 / y=0_{.}61 | 9100 |
| 61 | HTM2 | Bsp. 6 + 5% D4 | Bsp. 14 | 26.4 | 5.2 | x=0.31 / y=0.62 | 10300 |
| 62 # | HTM2 | Bsp. 6 + 5% D4 | Bsp. 37 | 25.1 | 5.1 | x=0.30 / y=0.61 | 11000 |
| 63 | HTM2 | Bsp. 6 + 5% D4 | Bsp. 40 | 24.6 | 5.1 | x=0.30 / y=0.62 | 10800 |

## Patentansprüche

1. Verbindung gemäß Formel (3), 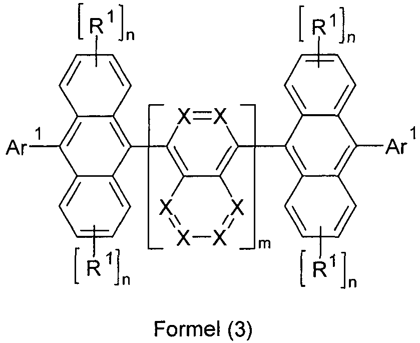 wobei für die verwendeten Symbole und Indizes gilt:
Ar¹ ist gleich oder verschieden bei jedem Auftreten eine kondensierte Aryl- oder Heteroarylgruppe mit 10 bis 16 aromatischen Ringatomen oder eine aromatische, gegebenenfalls überbrückte, Biarylgruppe, wobei die genannten Gruppen jeweils mit einem oder mehreren Resten R¹ substituiert sein können;
R¹ ist gleich oder verschieden bei jedem Auftreten H, F, Cl, Br, I, CN, NO₂, N(R²)₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch -C≡C-, Ge(R²)₂, Sn(R²)₂, C=O, C=S, C=Se, C=NR², -O-, -S-, -N(R²)- oder -CONR²- ersetzt sein können und wobei ein oder mehrere H-Atome durch F, Cl, Br, I, CN oder NO₂ ersetzt sein können, oder eine Aryloxy- oder Heteroaryloxygruppe mit 5 bis 24 aromatischen Ringatomen, die durch einen oder mehrere Reste R² substituiert sein kann, oder eine Kombination aus zwei, drei, vier oder fünf dieser Systeme;
R² ist bei jedem Auftreten gleich oder verschieden H oder ein Kohlenwasserstoffrest mit 1 bis 20 C-Atomen, welcher aliphatisch, aromatisch oder eine Kombination aus aliphatisch und aromatisch sein kann und worin auch ein oder mehrere H-Atome durch F ersetzt sein können; dabei können auch zwei oder mehrere Reste R² miteinander ein mono- oder polycyclisches, aliphatisches oder aromatisches Ringsystem bilden;
X steht gleich oder verschieden bei jedem Auftreten für CR¹ oder N;
n ist gleich oder verschieden bei jedem Auftreten 0, 1, 2, 3 oder 4;
m ist 1, 2, 3, 4 oder 5.

2. Verbindung nach Anspruch 1, **dadurch gekennzeichnet, dass** die in eckigen Klammern mit Index m stehende Gruppe in Formel (3) ausgewählt ist aus den Gruppen der Formeln (5) bis (10), 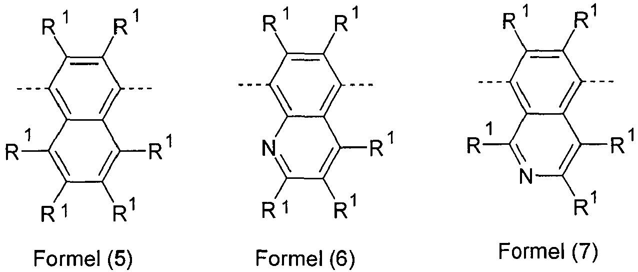 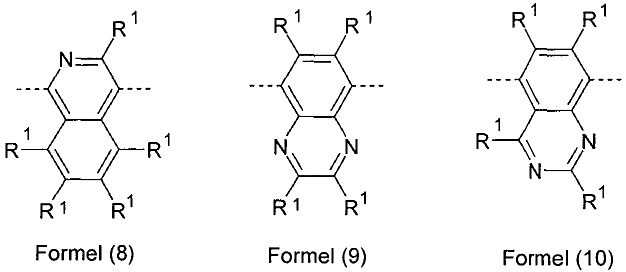 wobei die Symbole und Indizes dieselbe Bedeutung haben wie in Anspruch 1.

3. Verbindung nach Anspruch 1 oder 2, **dadurch gekennzeichnet, dass** die in eckigen Klammern mit Index m stehende Gruppe in Formel (3) 1,4-Naphthylen ist.

4. Verbindung nach einem oder mehreren der Ansprüche 1 bis 3, **dadurch gekennzeichnet, dass** das Symbol R¹, gleich oder verschieden bei jedem Auftreten, für H, F, eine geradkettige Alkyl- oder Alkoxygruppe mit 1 bis 6 C-Atomen oder eine verzweigte oder cyclische Alkyl- oder Alkoxygruppe mit 3 bis 10 C-Atomen, wobei jeweils ein oder mehrere CH₂-Gruppen durch-O-, -S- oder -N(R²)-ersetzt sein können und wobei jeweils ein oder mehrere H-Atome durch F ersetzt sein können, oder eine Kombination aus zwei oder drei dieser Systeme steht.

5. Verbindung nach einem oder mehreren der Ansprüche 1 bis 4, **dadurch gekennzeichnet, dass** der Index n für 0, 1 oder 2 steht, bevorzugt für 0 oder 1.

6. Verbindung nach einem oder mehreren der Ansprüche 1 bis 5, **dadurch gekennzeichnet, dass** der Index m für 1, 2 oder 3 steht, bevorzugt für 1 oder 2.

7. Verbindung nach einem oder mehreren der Ansprüche 1 bis 6, **dadurch gekennzeichnet, dass** die beiden Gruppen Ar¹ gleich sind und gleich substituiert sind.

8. Verfahren zur Synthese von Verbindungen nach einem oder mehreren der Ansprüche 1 bis 7, **dadurch gekennzeichnet, dass** die Bindungen zwischen dem Anthracen und den Gruppen Ar¹ bzw. Ar² durch Suzuki-Kupplung geknüpft werden.

9. Verbindung gemäß Formel (11), 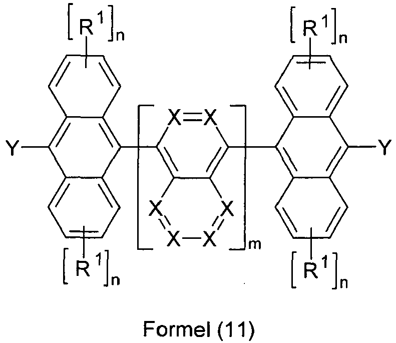 wobei gilt:
Y ist, gleich oder verschieden bei jedem Auftreten, Chlor, Brom, Iod oder eine Gruppe der Formel OSO₂R³, insbesondere Brom;
R³ ist eine organische Gruppe mit 1 bis 20 C-Atomen, in der auch einzelne H-Atome durch Fluor ersetzt sein können;
die weiteren Symbole und Indizes haben die in Anspruch 1 aufgeführte Bedeutung.

10. Verwendung von Verbindungen nach einem oder mehreren der Ansprüche 1 bis 7 in organischen elektronischen Vorrichtungen, insbesondere in organischen Elektrolumineszenzvorrichtungen.

11. Organische elektronische Vorrichtung, enthaltend mindestens eine Verbindung nach einem oder mehreren der Ansprüche 1 bis 7, insbesondere ausgewählt aus organischen Elektrolumineszenzvorrichtungen (OLED, PLED), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), organischen Photorezeptoren oder organischen Laserdioden (O-Laser).

12. Organische Elektrolumineszenzvorrichtung nach Anspruch 11, enthaltend Anode, Kathode und mindestens eine emittierende Schicht und gegebenenfalls weitere Schichten, ausgewählt aus Lochinjektionsschicht, Lochtransportschicht, Elektronentransportschicht, Elektroneninjektionsschicht und/oder eine Charge-Generation Layer.

13. Organische Elektrolumineszenzvorrichtung nach Anspruch 11 oder 12, **dadurch gekennzeichnet, dass** die Verbindung nach einem oder mehreren der Ansprüche 1 bis 7 als Hostmaterial zusammen mit einem Dotanden eingesetzt wird oder dass sie als Emitter oder als Elektronentransportverbindung eingesetzt wird.

14. Verfahren zur Herstellung einer organischen Elektrolumineszenzvorrichtung nach einem oder mehreren der Ansprüche 11 bis 13, **dadurch gekennzeichnet, dass** mindestens eine Verbindung nach einem oder mehreren der Ansprüche 1 bis 7, gegebenenfalls zusammen mit einem Dotanden und/oder anderen Verbindungen, durch ein Sublimationsverfahren oder aus Lösung oder durch ein Druckverfahren aufgebracht werden.

## Claims

1. Compound of the formula (3) 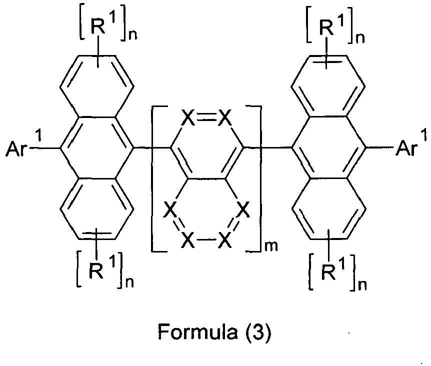 where the following applies to the symbols and indices used:
Ar¹ is, identically or differently on each occurrence, a condensed aryl or heteroaryl group having 10 to 16 aromatic ring atoms or an aromatic, optionally bridged biaryl group, where the said groups may each be substituted by one or more radicals R¹;
R¹ is, identically or differently on each occurrence, H, F, Cl, Br, I, CN, NO₂, N(R²)₂, a straight-chain alkyl, alkoxy or thioalkoxy group having 1 to 40 C atoms or a branched or cyclic alkyl, alkoxy or thioalkoxy group having 3 to 40 C atoms, each of which may be substituted by one or more radicals R², where one or more non-adjacent CH₂ groups may be replaced by -C≡C-, Ge(R²)₂, Sn(R²)₂, C=O, C=S, C=Se, C=NR², -O-, -S-, -N(R²)- or -CONR²- and where one or more H atoms may be replaced by F, Cl, Br, I, CN or NO₂, or an aryloxy or heteroaryloxy group having 5 to 24 aromatic ring atoms, which may be substituted by one or more radicals R², or a combination of two, three, four or five of these systems;
R² is on each occurrence, identically or differently, H or a hydrocarbon radical having 1 to 20 C atoms, which may be aliphatic, aromatic or a combination of aliphatic and aromatic and in which, in addition, one or more H atoms may be replaced by F; two or more radicals R² here may also form a mono- or polycyclic, aliphatic or aromatic ring system with one another;
X stands, identically or differently on each occurrence, for CR¹ or N;
n is, identically or differently on each occurrence, 0, 1, 2, 3 or 4;
m is 1, 2, 3, 4 or 5.

2. Compound according to Claim 1 **characterised in that** the group with index m in square brackets in formula (3) is selected from the groups of the formulae (5) to (10), 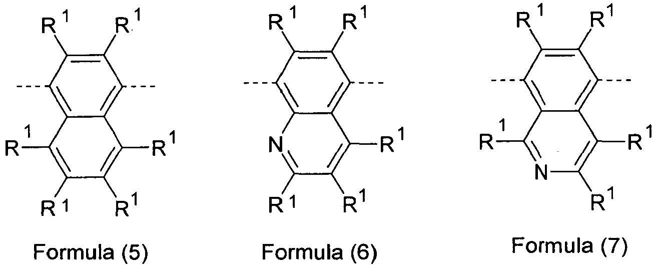 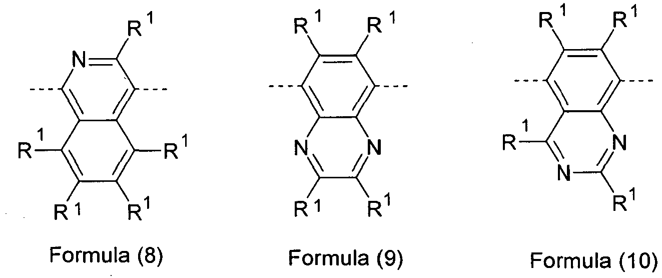 where the symbols and indices have the same meaning as in Claim 1.

3. Compound according to Claim 1 or 2, **characterised in that** the group with index m in square brackets in formula (3) is 1,4-naphthylene.

4. Compound according to one or more of Claims 1 to 3, **characterised in that** the symbol R¹, identically or differently on each occurrence, stands for H, F, a straight-chain alkyl or alkoxy group having 1 to 6 C atoms or a branched or cyclic alkyl or alkoxy group having 3 to 10 C atoms, where in each case one or more CH₂ groups may be replaced by -O-, -S- or -N(R²)- and where in each case one or more H atoms may be replaced by F, or a combination of two or three of these systems.

5. Compound according to one or more of Claims 1 to 4, **characterised in that** the index n stands for 0, 1 or 2, preferably for 0 or 1.

6. Compound according to one or more of Claims 1 to 5, **characterised in that** the index m stands for 1, 2 or 3, preferably for 1 or 2.

7. Compound according to one or more of Claims 1 to 6, **characterised in that** the two groups Ar¹ are identical and identically substituted.

8. Process for the synthesis of compounds according to one or more of Claims 1 to 7, **characterised in that** the bonds between the anthracene and the groups Ar¹ or Ar² are formed by Suzuki coupling.

9. Compound of the formula (11) 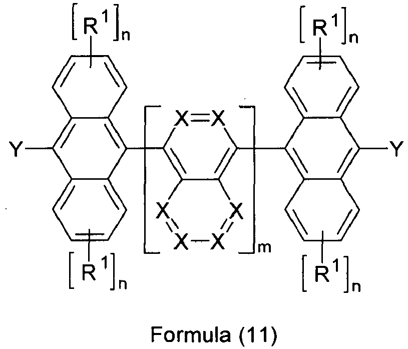 where:
Y is, identically or differently on each occurrence, chlorine, bromine, iodine or a group of the formula OSO₂R³, in particular bromine;
R³ is an organic group having 1 to 20 C atoms, in which, in addition, individual H atoms may be replaced by fluorine;
the other symbols and indices have the meaning given in Claim 1.

10. Use of compounds according to one or more of Claims 1 to 7 in organic electronic devices, in particular in organic electroluminescent devices.

11. Organic electronic device containing at least one compound according to one or more of Claims 1 to 7, selected, in particular, from organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), organic photoreceptors or organic laser diodes (O-lasers).

12. Organic electroluminescent device according to Claim 11, comprising anode, cathode and at least one emitting layer and optionally further layers selected from hole-injection layer, hole-transport layer, electron-transport layer, electron-injection layer and/or a charge-generation layer.

13. Organic electroluminescent device according to Claim 11 or 12, **characterised in that** the compound according to one or more of Claims 1 to 7 is employed as host material together with a dopant or **in that** it is employed as emitter or as electron-transport compound.

14. Process for the production of an organic electroluminescent device according to one or more of Claims 11 to 13, **characterised in that** at least one compound according to one or more of Claims 1 to 7, optionally together with a dopant and/or other compounds, are applied by a sublimation process or from solution or by a printing process.

## Revendications

1. Composé de la formule (3) : 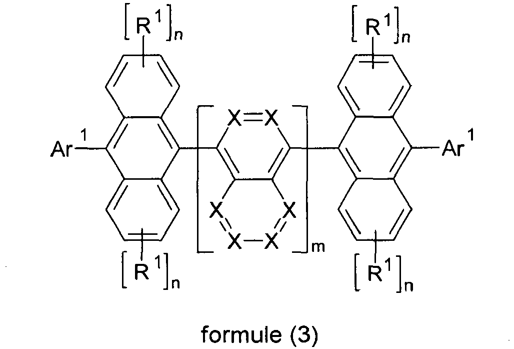 dans laquelle ce qui suit s'applique aux symboles et indices utilisés :
Ar¹ est, de manière identique ou différente pour chaque occurrence, un groupe aryle ou hétéroaryle condensé qui comporte 10 à 16 atomes de cycle aromatique, en option un groupe biaryle ponté, où lesdits groupes peuvent chacun être substitués par un radical ou plusieurs radicaux R¹ ;
R¹ est, de manière identique ou différente pour chaque occurrence, H, F, Cl, Br, I, CN, NO₂, N(R²)₂, un groupe alkyle, alcoxy ou thioalcoxy en chaîne droite qui comporte 1 à 40 atome(s) de C ou un groupe alkyle, alcoxy ou thioalcoxy ramifié ou cyclique qui comporte 3 à 40 atomes de C, dont chacun peut être substitué par un radical ou plusieurs radicaux R², où un ou plusieurs groupe(s) CH₂ non adjacents peut/peuvent être remplacé(s) par -C≡C-, Ge(R²)₂, Sn(R²)₂, C=O, C=S, C=Se, C=NR², -O-, -S-, -N(R²)- ou -CONR²- et où un ou plusieurs atome(s) de H peut/ peuvent être remplacé(s) par F, Cl, Br, I, CN ou NO₂, ou un groupe aryloxy ou hétéroaryloxy qui comporte 5 à 24 atomes de cycle aromatique, lequel peut être substitué par un radical ou plusieurs radicaux R², ou une combinaison de deux, trois, quatre ou cinq de ces systèmes ;
R² est, pour chaque occurrence, de manière identique ou différente, H ou un radical hydrocarbone qui comporte 1 à 20 atome(s) de C, lequel peut être aliphatique, aromatique ou une combinaison d'aliphatique et d'aromatique et où, en outre, un ou plusieurs atome(s) de H peut/peuvent être remplacé(s) par F ; deux radicaux R² ou plus peuvent ici également former un système de cycle aliphatique ou aromatique, monocyclique ou polycyclique l'un avec l'autre ou les uns avec les autres ;
X représente, de manière identique ou différente pour chaque occurrence, CR¹ ou N ;
n est, de manière identique ou différente pour chaque occurrence, 0, 1, 2, 3 ou 4 ;
m est 1, 2, 3, 4 ou 5.

2. Composé selon la revendication 1, **caractérisé en ce que** le groupe muni de l'indice m entre crochets dans la formule (3) est sélectionné parmi les groupes des formules (5) à (10) : 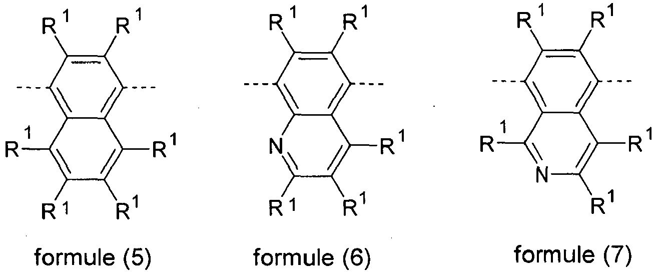 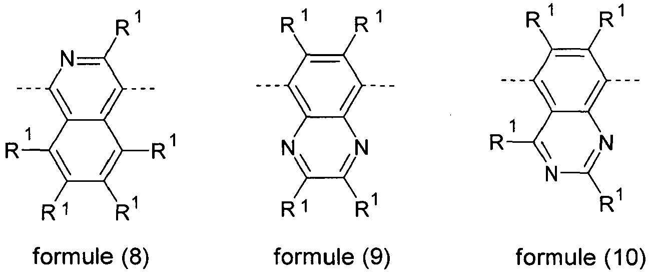 dans lesquelles les symboles et indices présentent la même signification que selon la revendication 1.

3. Composé selon la revendication 1 ou 2, **caractérisé en ce que** le groupe muni de l'indice m entre crochets dans la formule (3) est 1,4-naphthylène.

4. Composé selon une ou plusieurs des revendications 1 à 3, **caractérisé en ce que** le symbole R¹ représente, de manière identique ou différente pour chaque occurrence, H, F, un groupe alkyle ou alcoxy en chaîne droite qui comporte 1 à 6 atome(s) de C ou un groupe alkyle ou alcoxy ramifié ou cyclique qui comporte 3 à 10 atomes de C, où, dans chaque cas, un ou plusieurs groupe(s) CH₂ peut/peuvent être remplacé(s) par -O-, -S- ou -N(R²)- et où, dans chaque cas, un ou plusieurs atome(s) de H peut/peuvent être remplacé(s) par F, ou une combinaison de deux ou trois de ces systèmes.

5. Composé selon une ou plusieurs des revendications 1 à 4, **caractérisé en ce que** l'indice n représente 0, 1 ou 2, de préférence 0 ou 1.

6. Composé selon une ou plusieurs des revendications 1 à 5, **caractérisé en ce que** l'indice m représente 1, 2 ou 3, de préférence 1 ou 2.

7. Composé selon une ou plusieurs des revendications 1 à 6, **caractérisé en ce que** les deux groupes Ar¹ sont identiques et sont substitués de manière identique.

8. Procédé pour la synthèse de composés selon une ou plusieurs des revendications 1 à 7, **caractérisé en ce que** les liaisons entre l'anthracène et les groupes Ar¹ ou Ar² sont formées au moyen d'un couplage de Suzuki.

9. Composé de la formule (11) : 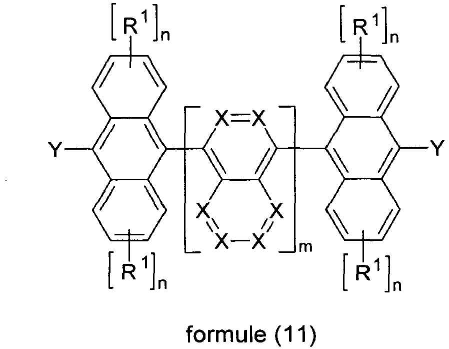 dans laquelle :
Y est, de manière identique ou différente pour chaque occurrence, chlore, brome, iode ou un groupe de la formule OSO₂R³, en particulier brome ;
R³ est un groupe organique qui comporte 1 à 20 atome(s) de C, où, en outre, des atomes de H individuels peuvent être remplacés par fluor ;
les autres symboles et indices présentent la signification qui a été donnée selon la revendication 1.

10. Utilisation de composés selon une ou plusieurs des revendications 1 à 7 dans des dispositifs électroniques organiques, en particulier dans des dispositifs électroluminescents organiques.

11. Dispositif électronique organique contenant au moins un composé selon une ou plusieurs des revendications 1 à 7, sélectionné, en particulier, parmi les dispositifs électroluminescents organiques (OLED, PLED), les transistors à effet de champ organiques (O-FET), les transistors à film mince organiques (O-TFT), les transistors à émission de lumière organiques (O-LET), les circuits intégrés organiques (O-IC), les cellules solaires organiques (O-SC), les dispositifs à extinction de champ organiques (O-FQD), les photorécepteurs organiques ou les diodes laser organiques (O-laser).

12. Dispositif électroluminescent organique selon la revendication 11, comprenant une anode, une cathode et au moins une couche d'émission et en option, d'autres couches qui sont sélectionnées parmi une couche d'injection de trous, une couche de transport de trous, une couche de transport d'électrons, une couche d'injection d'électrons et/ou une couche de génération de charges.

13. Dispositif électroluminescent organique selon la revendication 11 ou 12, **caractérisé en ce que** le composé selon une ou plusieurs des revendications 1 à 7 est utilisé en tant que matériau hôte en association avec un dopant ou **en ce qu'**il est utilisé en tant qu'émetteur ou en tant que composé de transport d'électrons.

14. Procédé pour la production d'un dispositif électroluminescent organique selon une ou plusieurs des revendications 11 à 13, **caractérisé en ce qu'**au moins un composé selon une ou plusieurs des revendications 1 à 7, en option en association avec un dopant et/ou d'autres composés, est appliqué au moyen d'un procédé de sublimation ou à partir d'une solution ou au moyen d'un procédé d'impression.
